# EUROPÄISCHE PATENTSCHRIFT

(11) **EP 1 165 769 B1**
(45) Veröffentlichungstag und Bekanntmachung des Hinweises auf die Patenterteilung: **25.09.2002**
(21) Anmeldenummer: 00912356.3
(22) Anmeldetag: 03.02.2000
(51) Int. Cl.: C12N 15/10, C12Q 1/68, B01L 7/00

(54) **VERFAHREN ZUR AUFTRENNUNG VON IN LÖSUNG BEFINDLICHEN DOPPELSTRÄNGIGEN NUKLEINSÄUREN IN EINZELSTRÄNGIGE NUKLEINSÄUREN**
METHOD FOR FRACTIONATING DOUBLE-STRANDED NUCLEIC ACIDS IN SOLUTIONS IN ORDER TO OBTAIN SINGLE-STRANDED NUCLEIC ACIDS
PROCEDE DE SEPARATION D'ACIDES NUCLEIQUES A DOUBLE BRIN EN SOLUTION PERMETTANT D'OBTENIR DES ACIDES NUCLEIQUES A UN SEUL BRIN

(30) Priorität: 12.02.1999 DE 19907470
(43) Veröffentlichungstag der Anmeldung: 02.01.2002
(73) Patentinhaber: Biotix GmbH, 14943 LuckenWalde (DE)
(72) Erfinder: KRAHN, Thomas, D-12249 Berlin (DE)
(74) Vertreter: Jungblut, Bernhard Jakob, Dr
(86) Internationale Anmeldenummer: DE0000338
(87) Internationale Veröffentlichungsnummer: WO00047732

(56) Entgegenhaltungen:
- WO-A-94/29484
- WO-A-98/06876
- WO-A-99/39008
- FR-A- 2 654 000

## Beschreibung

Die Erfindung betrifft ein Verfahren zur Auftrennung von in Lösung befindlichen doppelsträngigen Nukleinsäuren in einzelsträngige Nukleinsäuren. - Der Begriff der Nukleinsäuren umfaßt neben DNA und RNA auch PNA. Mit diesem Begriff sind weiterhin natürliche Nukleinsäuren, i.e. Nukleinsäuren voller Länge, umfaßt, als auch Nukleinsäurefragmente. Fragmente bezeichnet dabei Teile einer (natürlich oder gentechnisch hergestellten) Nukleinsäure bzw. synthetisierte Nukleinsäuren. Typische Basenpaarzahlen von Fragmenten liegen beispielsweise im Bereich von kleiner als 10 bis zu über 2000.

Verfahren der vorstehenden Art werden z.B. in dem Bereich der Amplifikation bzw. Vervielfältigung von Nukleinsäurestrukturen oder bei der Hybridisierung zu untersuchender Nukleinsäuren mit bekannten Nukleinsäuren zum Zwecke des Erhalts von Informationen über die Sequenz der unbekannten Nukleinsäuren (Stichworte: DNA-Hybridisierungstechniken, DNA Chips) benötigt. Grundlage vieler gentechnischer Analyseverfahren ist die selektiv arbeitende PCR (Polymerase chain reaction), eine einfache Vervielfältigungsmethode für gezielt aussuchbare DNA's oder DNA-Fragmente in einer Reaktionskammer (siehe auch US-A-4,683,202) oder auch in situ, beispielsweise in einem Gewebeschnitt. Nach der Einführung temperaturstabiler Polymerasen hat diese Methode weite Verbreitung in gentechnischen Laboratorien gefunden.

PCR ist insbesondere die gezielte Vervielfältigung eines präzise aus dem Genom ausgesuchten relativ kurzen Segments der zweisträngigen DNA in einfachen Temperaturzyklen. Die Auswahl des DNA-Segments erfolgt durch ein sogenanntes Paar von Primern, zweier DNA-Stücke mit je etwa 20 Basen Länge, die die beiderseitigen Enden des ausgesuchten DNA-Segments codieren. Die Vervielfältigung erfolgt durch ein Enzym namens Polymerase. Die PCR-Reaktion läuft in wäßriger Lösung ab, in der wenige Moleküle der Ausgangs-DNA und genügende Mengen an Polymerase, Primern, Triphosphaten der vier Nukleotide, Aktivatoren und Stabilisatoren vorhanden sind. In jedem Temperaturzyklus wird zunächst die Doppelhelix der DNA bei etwa 95 °C "aufgeschmolzen", wobei die beiden Stränge voneinander getrennt werden. Bei etwa 45 °C werden dann die Primer an eine genau passend komplementäre Nukleotidfolge der DNA-Einzelstränge angelagert ("Hybridisierung"). Bei 72 °C wird die Doppelhelix durch den Anbau der komplementären Nukleotide an ein Wachstumsende der Primer durch eine besonders temperaturfeste Polymerase (Taq-Polymerase) wieder zu einer neuen Doppelhelix vervollständigt. Dadurch wird das ausgewählte DNA-Segment zwischen den Primern im Prinzip verdoppelt. In dreißig Zyklen werden also aus einem einzigen Doppelstrang der DNA als Ausgangsmaterial rund eine Milliarde DNA-Segmente erzeugt, deren beide Enden mit den Primern identisch sind.

Die Vervielfältigung der DNA erfolgt bei den heutigen PCR Geräten schon relativ schnell im Vergleich zu den früher benutzten Klonierungsmethoden in lebenden Zellen. Bei optimiertem Steuerprogramm kann innerhalb drei Stunden mit einem handelsüblichen PCR-Gerät aus aufgeschlossenem Zellmaterial ein etwa 400 Basenpaare langes DNA-Fragment amplifiziert werden, so daß sich die DNA Konzentration deutlich über die Nachweisgrenze (im Agarosegel unter UV Bestrahlung und Färbung mit Ethidiumbromid) erhöht.

Das Schmelzen der DNA erfolgte bisher bei einer Temperatur, die knapp über der empirisch ermittelten Schmelztemperatur liegt. Untersuchungen haben gezeigt, daß eine halbe Sekunde Erhitzung auf diese Temperatur für eine vollständige Trennung aller Doppelhelix-Strukturen genügt. Die Hybridisierung braucht ebenfalls nicht lange, wenn die Primer in genügender Konzentration vorhanden sind. Bei optimaler Konzentration sind etwa ein bis zwei Sekunden ausreichend. Die Vervollständigung der DNA hat eine sehr hohe Geschwindigkeit: Pro Sekunde können unter optimalen Temperatur- und Konzentrationsbedingungen 50 bis 100 Basen durch eine spezielle Polymerase angebaut werden (C. R. Newton und A. Graham: PCR; Spektrum Akademischer Verlag; Heidelberg, Berlin, Oxford, 1994; Seite 31). Da im allgemeinen nur DNA Segmente von bis zu 400 Basen Länge für die Analysen benötigt werden, reichen zehn Sekunden gut für die Verlängerung aus.

Die Zeitdauer für einen Temperaturzyklus hängt somit größtenteils von der Aufheiz- und Abkühlgeschwindigkeit ab, die wiederum vom Flüssigkeitsvolumen, von den Gefäßdimensionen und von der Wärmeleitfähigkeit der Gefäßwände und der Reaktionslösung abhängt. Auf jeder Temperaturstufe sind im Prinzip nur wenige Sekunden vonnöten, teilweise sogar weniger.

Es ist bekannt durch Verringerung des Probenvolumens, Vergrößerung der spezifischen Oberfläche und durch pneumatisches Anlegen eines Kühlblocks die Übergangszeiten zum folgenden Temperaturniveau so kurz wie möglich zu halten (DE 197 17 085 A 1). Dies ist jedoch nur mit erheblichem mechanischen und mikrosystemtechnischen Aufwand möglich.

Aus der Literaturstelle WO 98/00562 ist es bekannt, den Aufschmelzprozeß mittels eines elektrischen Feldes durchzuführen, welches im Zuge des Reaktionsverlaufes abgeschaltet oder invertiert wird.

Aus der Literaturstelle WO 96/41864 ist es bekannt, eine zum Aufschmelzen erforderliche Temperatur in der Lösung mittels Einstrahlung von IR- und UV-Licht einzustellen.

Aus der Literaturstelle WO 98/06876 ist es bekannt, den Aufheizvorgang zum Aufschmelzen dadurch durchzuführen, daß ein in der Lösung befindliches Festkörpersubstrat mittels Einstrahlung von elektromagnetischen Wellen aufgeheizt wird. Die zum Einsatz kommenden elektromagnetischen Wellen haben Frequenzen unterhalb von 1,07 GHz.

WO 94 29 484 beschreibt ein Verfahren zur Auftrennung von doppelsträngigen Nulkleinsäuren unter Verwendung von elektromagnetischer Strahlung und eine dafür geeignete Vorrichtung. Dabei werden zwei spezielle Primer ("magnetic primer") verwendet.

Eine hohe Geschwindigkeit der PCR ist insbesondere aus den folgenden Gründen erwünscht. Im Rahmen des "Human Genome Project" wird versucht, die Sequenz des menschlichen Genoms zu entschlüsseln. Da dieses Genom etwa 3 Milliarden Basenpaare groß ist, und bisher (Stand: Oktober 1998) erst etwa 6,8% davon sequenziert wurde, sprechen jüngste Hochrechnungen davon, daß noch etwa 25 Jahre benötigt werden, um das Projekt abzuschließen. Die Förderprogramme für dieses Projekt gehen allerdings davon aus, daß bereits in sechs Jahren alle Basen sequenziert sein sollen. Da die PCR ein wesentlicher Bestandteil der Sequenzierung ist und alle anderen Schritte der Sequenzierung heute wesentlich schneller ablaufen, hängt der Erfolg des Projekts insbesondere von der Geschwindigkeit der PCR ab. Weiterhin existieren PCR Anwendungen die zur Erkennung von bestimmten Tumorzellen in menschlichem Gewebe eingesetzt werden können. Sollen solche Analysen während einer Operation am offenen Patienten durchgeführt werden, dann wird eine maximale Analysendauer von 10 Minuten gefordert. Die bisher schnellsten beschriebenen PCR Geräte (A. T. Woodley et. Al., "Functional Integration of PCR Amplification and Capillary Electrophoresis in a Microfabricated DNA Analysis Device", Anal. Chem. 68, 4081, Dezember 1996) benötigen jedoch mindestens 15 Minuten bei einem Programm mit 30 Zyklen. Die Zeit, die man zum Aufschließen der Zellen und zur Freilegung der DNA benötigt ist dabei noch nicht eingerechnet. Schließlich wird PCR beispielsweise auch bei der Selektion von gegen vorgegebene Zielstoffe affinen Aptameren bzw. Ribozymen aus hochkomplexen DNA-Bibliotheken (10¹⁵ und mehr) eingesetzt.

Ein weiteres Problem der bekannten PCR Verfahren ist die irreversible Zerstörung der Polymerase durch die hohe Temperatur während des Aufschmelzens. Obwohl es heute schon sehr gut auf Temperaturstabilität optimierte Polymerasen gibt, sinkt die Leistungsfähigkeit der Enzyme bei jedem Zyklus beträchtlich ab. Die Halbwertszeit der Aktivität üblicher Polymerasen (von *Thermus aquaticus*) bei 95 °C beträgt 40 Minuten (C. R. Newton und A. Graham: PCR; Sektrum Akademischer Verlag; Heidelberg, Berlin, Oxford, 1994; Seite 31). Gerade während der letzten Zyklen wird aber eine sehr hohe Aktivität der Polymerase gefordert, da bereits sehr viele DNA Amplifikate erzeugt wurden, die alle gleichzeitig verlängert werden sollen. Es muß daher eine entsprechend höhere Menge des teuren Enzyms eingesetzt werden. Einige PCR Verfahren begegnen diesem Problem durch eine sukzessive Verlängerung der Elongationszeiten in jedem Zyklus. Ein Polymerase-Molekül kann dann mehrmals hintereinander an unterschiedlichen Stellen während eines Zyklus eingesetzt werden. Dies erhöht jedoch wiederum die Gesamtdauer der PCR. Es wäre wünschenswert, wenn eine Optimierung der Verlängerungsgeschwindigkeit der Polymerasen gelänge, anstatt die Temperaturstabilität der Enzyme weiter verbessern zu müssen.

Den bekannten Technologien ist gemeinsam, daß der Bedarf an elektrischer Energie aufgrund der notwendigen Heiz- und Kühlprozesse relativ hoch ist mit der Folge, daß entsprechende Stromversorgungseinheiten vorgesehen sein müssen. Dies macht Geräte des Standes der Technik voluminös und schwer und erschwert einen vor-Ort Einsatz außerhalb eines speziell vorgesehenen Labors.

Daher liegt der Erfindung das technische Problem zugrunde, ein Verfahren anzugeben, mittels welchem innerhalb kürzester Zeit eine doppelsträngige Nukleinsäure in Einzelstränge zerteilt werden kann. Insbesondere ist es wünschenswert, die Zykluszeit für die PCR Amplifikation von Nukleinsäuren zu beschleunigen und auf wenige Sekunden zu reduzieren. Ebenso wünschenswert ist es, die für die Polymerase schädliche Erhitzung auf hohe Temperaturen zu vermeiden und so die PCR effektiver werden zu lassen und gleichzeitig teures Polymerase Enzym einzusparen. Schließlich wäre eine Verringerung des Bedarfes an elektrischer Energie zum Zwecke eines mobilen Einsatzes, beispielsweise mit Batterien, wünschenswert Zur Lösung dieses technischen Problems lehrt die Erfindung, daß die Zerlegung der Nukleinsäuren in Einzelstränge durch Einstrahlung von elektromagnetischen Wellen durchgeführt wird, wobei die Frequenz und die Intensität der elektromagnetischen Wellen mit der Maßgabe ausgewählt sind, daß die Wasserstoffbrückenbindungen zwischen Nukleotiden einer doppelsträngigen Nukleinsäure durch unmittelbare Wechselwirkung der elektromagnetischen Strahlung mit der doppelsträngigen Nukleinsäure gelöst werden.

Die Erfindung beruht auf den folgenden Erkenntnissen. Die genaue Struktur, die Bindungslängen, die Bindungswinkel und die Bindungsenergie der Wasserstoffbrückenbindungen zwischen den Nukleinsäure bzw. DNA Strängen und die molekularen Massenanteile der Nukleotide sind sehr genau bekannt. Wenn dann die Frequenzen der elektromagnetischen Wellen zudem so ausgewählt sind, daß die Wellen mit dem Medium, i.e. Reaktionspuffer bzw. seinen mengenmäßigen Hauptkomponenten, eine möglichst geringe Wechselwirkung aufweisen, kann eine (unerwünschte) thermische Aufheizung des Gesamtgemisches vermieden werden. Es versteht sich, daß die Frequenzen auch so gewählt sein sollten, daß-keine störende Absorption durch die Polymerasen stattfindet.

Zum Schmelzen der Nukleinsäure muß dem Molekül Energie hinzugefugt werden, deren Betrag mindestens so hoch ist, wie die Bindungsenergie der Wasserstoffbrücke. Dies geschieht in der vorstehend beschriebenen PCR durch Temperaturerhöhung. Die für die Erfindung wesentliche Erkenntnis ist, daß Nukleinsäuremoleküle wegen ungleicher Verteilung elektrischer Ladungen durch elektromagnetische Wechselfelder zum Schwingen angeregt werden können. Die Resonanzfrequenzen können durch absortionsspektroskopische Messungen gelöster Nukleinsäuren im Millimeterwellen- und Submillimeterwellenbereich (10 - 500 GHz) bzw. im fernen IR (0,5 bis 10 THz) ermittelt werden. Einige dieser Schwingungszustände bewirken eine radiale periodische Valenzschwingung zwischen durch Wasserstoffbrückenbindungen miteinander verknüpften Nukleotiden verschiedener Stränge. Wenn im Bereich dieser Resonanzfrequenzen mittels eines Hochfrequenzgenerators mit ausreichender Leistung Energie eingestrahlt wird, kann dies zu einer Lösung der Wasserstoffbrückenbindungen und folglich einer Vereinzelung der beiden Stränge einer doppelsträngigen Nukleinsäure führen. Das millimeterwelleninduzierte Auftrennen in die Einzelstränge kann aufgrund des hyperchromen Effekts der Nukleinsäuren mit Hilfe von Messung der UV-Absorption oder -Transmission (Wellenlänge: 260 nm) nachgewiesen werden. Wird der Hochfrequenzgenerator abgeschaltet (oder wird die Nukleinsäure in einen Bereich geringer elektrischer Feldstärke transportiert), dann rehybridisieren die einzelsträngigen Nukleinsäuren selbstständig zur doppelsträngigen Nukleinsäure. Es ist somit möglich, durch gezielte Einstrahlung elektromagnetischer Wellen mit definierter Frequenz, insbesondere Nukleinsäure-Resonanzfrequenzen, welche abseits von Resonanzen des Mediums bzw. der Polymerasen liegen, selektiv eine Trennung der Nukleinsäurestränge voneinander zu bewirken, und zwar ohne eine beim Stand der technik festzustellende unerwünschte Erwärmung des Puffers und insbesondere der Polymerasen. Dies wird (neben der nicht beobachteten Erwärmung) auch dadurch belegt, daß die Trennung nur wenige Milliwatt, beispielsweise 0,1 bis 1000 Milliwatt, insbesondere 1 bis 100 Milliwatt, erfordert bei geeigneter Anpassung der Expositionsvorrichtung und Positionierung des Reaktionsgefäßes.

In diesen Zusammenhängen ist aber auch anzumerken, daß die Prozesse der Auftrennung der doppelsträngigen Nukleinsäure nicht auf die direkte Trennung der Wasserstoffbrückenbindungen beschränkt sein müssen. Eine Rolle können auch Schwingungszustände des Gesamtmoleküls oder von Teilen daraus eine Rolle spielen, da diese Schwingungszustände die Wasserstoffbrückenbindungen jedenfalls in angeregte Zustände dann überführen werden, wenn die Schwingungszustände zu beispielsweise sterischen Spannungen im Bereich der Wasserstoffbrückenbindungen führen. Es ist also auch möglich, daß die Anregung von Schwingungszuständen der Stränge zu einer Auftrennung eines Doppelstrangs führen können. Daher meint der Ausdruck der unmittelbaren Wechselwirkung der Strahlung mit der doppelsträngigen Nukleinsäure neben Anregungen der Wasserstoffbrückenbindungen auch die Anregung von anderen Bindungen der Nukleinsäure, welche zumindest zu einer Anregung, i.e. Schwächung der Bindungskräfte, der Wasserstoffbrückenbindung führt. Anregung meint im wesentlichen die Anregung von Dehnschwingungen und/oder Biegeschwingungen molekularer Bindungen. Im Falle von Schwingungen des Molekülgerüstes einer oder beider Stränge können sich dabei auch übergeordnete Strukturen, insbesondere Wellenphänomene und Scherkräfte bilden.

Da in einer Nukleinsäure immer mindestens ein gebundenes Stickstoffatom beteiligt ist, haben jedenfalls die Wasserstoffbrücken immer eine niedrigere Bindungskraft als die des umgebenden Wassers, welches nur stärker gebundene Sauerstoffatome als Donoren besitzt. Es ist somit bei geeigneter Wahl der Einstrahlfrequenz möglich, gezielt die Nukleinsäure zu schmelzen, ohne gleichzeitig das Umgebungswasser in störender Weise zu erhitzen. Aber auch Frequenzen oberhalb der Resonanzfrequenz des Wasser sind einsetzbar, da beispielsweise im Bereich von 0,5 bis 2,0 THz charakterische Resonanzen des Molekulgerüsts ("Atmung") beobachtet werden. Auch bei Wahl solch hoher (auf eine geeignete Resonanzfrequenz des Molekülgerüsts abgestimmter) Frequenzen erfolgt letztendlich eine Aufspaltung der Wasserstoffbrückenbindungen aufgrund der dabei entstehenden "Spannungen" im Bereich der Wasserstoffbrücken. Es ist auch möglich, sowohl im Bereich der Wasserstoffbrücken-Resonanzen als auch im Bereich der Gerüst-Resonanzen einzustrahlen.

Im Rahmen der Erfindung bevorzugt ist es, wenn die Freqenz der elektromagnetischen Strahlung im Bereich von 10 GHz bis 2THz, vorzugsweise 10 bis 250 GHz, höchstvorzugsweise 30 bis 79 GHz, beispielsweise 45 bis 53 GHz, eingestellt ist mit der Maßgabe, daß die eingestellte Frequenz zumindest teilweise solche Schwingungszustände der Nukleinsäure anregt, welche in einer Trennung ausschließlich der Wasserstoffbrückenbindungen resultieren, nicht jedoch Bindungen einer Polymerase spaltet (dies schließt geringfügige Zersetzungsraten von bis zu 10%, vorzugsweise weniger als 2%, der Rate bei Erwärmung auf 95°C ein) oder zu einer Temperaturerhöhung eines Mediums führt (dies schließt unwesentliche Temperaturerhöhungen eines nicht thermostatisierten Mediums von bis zu 20°C / min., vorzugsweise von weniger als 5°C / min. ein). Dies ist der Frequenzbereich, in welchem experimentell geeignete Resonanzen gefunden wurde, um doppelsträngige DNA bei Temperaturen unterhalb der DNA-Schmelztemperatur, bis hinunter zu 20°C, zu schmelzen.

Von selbstständiger Bedeutung im Rahmen der Erfindung ist die Verwendung des erfindungsgemäßen Verfahrens in einem Verfahren zur in vitro Amplifikation von Nukleinsäuren mit den folgenden Verfahrenstufen: a) Zerlegung der Nukleinsäuren in Einzelstränge, b) Anhybridisierung von Primern an die Einzelstränge aus Stufe a), c) Verlängerung der in Stufe b) anhybridisierten Primer durch (Desoxy-) Ribonukleosidtriphosphate mittels Einsatz einer Polymerase, d) Rückführung der in Stufe c) erhaltenen Nukleinsäuren in die Stufe a), wobei die Stufen a) - d) so oft wiederholt werden, bis ein vorgegebener Amplifikationsfaktor erreicht ist und wobei die elektromagnetische Strahlung in Stufe a) eingestrahlt wird. Bevorzugt ist es, wenn auf Nukleinsäure-Synthese-Geschwindigkeit optimierte Polymerasen in Stufe b) eingesetzt werden. Diese brauchen nämlich nicht mehr die beim Stand der Technik erforderliche Temperaturbeständigkeit aufzuweisen.

Gegenstand der Erfindung ist weiterhin eine Vorrichtung zur Durchführung eines Verfahrens nach einem der Ansprüche 1 bis 4 mit einer Reaktionskammer zur Aufnahme einer Lösung mit Nukleinsäuren, mit einer Vorrichtung zur Erzeugung elektromagnetischer Wellen sowie einem Antennenelement zur Abstrahlung der elektromagnetischen Strahlung, wobei das Antennenelement unmittelbar bei der Reaktionskammer angeordnet ist und wobei zumindest eine Betriebsfrequenz der Vorrichtung zur Erzeugung elektromagnetischer Strahlung im Bereich von 10 bis 250 GHz, vorzugsweise 30 bis 79 GHz, und/oder 0,5 bis 2,0 THz liegt. - Der Ausdruck der Antennenelemente bezeichnet jegliche Bauelemente, von denen elektromagnetische Wechselfelder emittiert werden können. Diese können beispielsweise um die Reaktionskammer herum oder auch nur an einer Seite außenseitig angeordnet sein. Denkbar ist auch der Einbau eines Antennenelements im Reaktionskammerinneren. Eine besonders vorteilhafte Ausführungsform für den unteren genannten Frequenzbereich ist dadurch gekennzeichnet, daß die Reaktionskammer innerhalb eines als Antennenelement funktionierenden elektrischen Resonanzkörpers, insbesondere eines Topfkreises oder Hohlleiters, angeordnet ist.

Die Reaktionskammer kann auf verschiedene Weisen ausgebildet sein. So kann die Reaktionskammer als Rührkessel-Reaktor ausgebildet sein. Dies meint, daß die Reaktionslösung stationär bleibt und ggf. agitiert wird. Es ist aber auch möglich, die Reaktionskammer als Rohrreaktor auszubilden, wobei im Inneren des Rohrreaktors und in Richtung der Längserstreckung des Rohrreaktors stehende elektromagnetische Wellen erzeugbar sind. In einem Rohrreaktor ist die Reaktionslösung mobil und strömt in Richtung der Längserstreckung des Reaktors. Dabei ist der Reaktionsfortschritt bzw. sind die Reaktionsstufen eine Funktion des Ortes (in erster Näherung eindimensional entlang der Längserstreckung). Eine weitere bevorzugte Ausführungsform der Reaktionskammer ist eine (ggf. beidseitig geschlossene) Kapillare, wobei die Kapillare hinsichtlich ihrer Längserstreckung zumindest zum Teil in Richtung des elektrischen Feldvektors der elektromagnetischen Strahlung ausgerichtet ist.

Mit der Erfindung wird erreicht, daß bei der PCR die Aufheiz- und Abkühlungsperiode entfällt, so daß sich die Zykluszeit und damit die Gesamtdauer des Amplifikationsvorgangs im Vergleich zur vorbekannten PCR erheblich verkürzt. Gleichzeitig wird die zur Vervielfachung notwendige Polymerase praktisch nicht durch Hitzedenaturierung geschädigt, so daß eine kleinere Menge des teuren Enzyms eingesetzt werden kann. Dies beruht letztendlich darauf, daß Resonanzen der Polymerase typischerweise nicht bei gleichen Frequenzen wie die Resonanzen zwischen zwei Nukleinsäure Strängen oder Molekül-Resonanzen in einem Strang liegen. Wesentlich ist hierbei also letztendlich, daß der Aufschmelzvorgang erfindungsgemäß nicht durch Temperaturerhöhung, sondern durch Einstrahlung elektromagnetischer Wellen mit einer definierten Nukleinsäure- bzw. Wasserstoffbrückenbindungsspezifischen Frequenz erfolgt.

Folgend werden Details der Erfindung sowie weitere mögliche Ausführungsformen der Erfindung näher erläutert.

Bei der PCR durchläuft das Reaktionsgemisch im einzelnen die folgenden physikalischen und chemischen Zustandsänderungen:
a) Das Reaktionsgemisch wird mit elektromagnetischen Wellen einer oder mehrerer definierter Frequenzen mit ausreichender Leistung bestrahlt. Doppelsträngige DNA geht dabei in einzelsträngige DNA über. Die Temperatur des Reaktionsgemisches wird dabei von den. elektromagnetischen Schwingungen nicht wesentlich beeinflußt.
b) Die Einstrahlung der elektromagnetischen Wellen wird beendet und das Reaktionsgemisch wird auf eine geeignete Temperatur geregelt, die unterhalb der Anlagerungstemperatur der Primer liegt. Die Primer können sich an den komplementären Stellen der einzelsträngigen DNA anlagern.
c) Das Reaktionsgemisch wird auf eine Temperatur geregelt, bei der die Polymerase mit optimaler Geschwindigkeit den komplementären Strang mit Nukleotiden auffüllt. Diese drei Stufen können so oft wie gewünscht wiederholt werden. Das entstandene doppelsträngige Reaktionsprodukt wird dabei wieder in (a) eingesetzt.

In der Praxis ist es möglich, für die Verfahrensstufen b) und c) die gleiche Temperatur, beispielsweise im Bereich von 20°C bis 80 °C, einzustellen. Der Verlauf der Gesamtreaktion ist dann isotherm. Dies ermöglicht eine beachtliche apparative Vereinfachung gegenüber PCR gemäß dem Stand der Technik, bei welchem mit hoher Präzision und folglich hohem technischen Aufwand unterschiedliche Temperaturstufen angefahren und eingeregelt werden müssen, und dies zudem möglichst schnell. Mit anderen Worten ausgedrückt, bei isothermer Verfahrensweise gemäß der Erfindung entfallen zudem die zeitaufwendigen Heiz- und Kühlphasen zwischen den verschiedenen Verfahrenstufen.

Das Reaktionsgemisch setzt sich dabei aus den üblichen Komponenten zusammen, wie sie bei der konventionellen PCR oder deren Weiterentwicklungen benutzt werden. Dies sind mindestens ein geeignetes Lösungsmittel, eine sehr kleine Menge Nukleinsäure als Matritze, ein oder mehrere geeignete Oligonukleotide als Primer, Desoxynukleotidtriphosphate aller in der Matritze zwischen den Primern liegenden Nukleinbasen, Eine Substanz, welche die Kettenverlängerung katalysiert (im übrigen Text Polymerase genannt).

Im Prinzip können alle Varianten, Abarten und Weiterentwicklungen der PCR (insbesondere die Sequenzierreaktion mit der Kettenabbruchmethode nach Sanger) mit diesem Verfahren durchgeführt werden. Gegebenenfalls müssen oder können entsprechende Anpassungen durchgeführt werden, um die Vorteile der Erfindung optimal ausnützen zu können.

Der wichtigste Nutzen der Erfindung ist darin begründet, daß in der Praxis die Temperatur des Reaktionsgemisches nie die Schmelztemperatur der beteiligten Nukleinsäurepaarungen erreicht oder gar überschreitet. In der Regel kann eine PCR bei Temperaturen des Reaktionsgemisches von nicht mehr als 75°C, insbesondere nicht mehr als 60°C, durchgeführt werden. Es werden folglich keine speziellen hitzeresistenten Polymerasen mehr benötigt. Es liegt nahe, die eingesetzten Polymerasen so auszuwählen bzw. zu konstruieren, daß sie möglichst schnell die Elongation des neu aufzubauenden DNA Stranges synthetisieren. So wird der bisher langsamste und damit geschwindigkeitsbestimmende Schritt der PCR deutlich verkürzt. Natürlich spart man auch die Aufheiz- und die Abkühlzeiten für die Auftrennung der beiden DNA Stränge ein, da die elektromagnetischen Wellen unmittelbar auf die DNA einwirken. Bei der konventionellen PCR muß sich zuerst der Heizblock und dann das Reaktionsgefäß und das umgebende Lösungsmittel aufheizen, bevor die Wärme an die DNA Moleküle weitergeleitet wird.

Die elektromagnetischen Wellen werden in der Praxis von einem elektronischen Hochfrequenzgenerator erzeugt und mit einer Abstrahlvorrichtung (Antenne) auf das dicht positionierte Reaktionsgemisch gerichtet. Als HochfrequenzGeneratoren kommen z.B. Gunn-, Impatt-, Backward-Waveoder Klystron-Oszillatoren in Frage. Eine zu tief liegende (Grund-) Frequenz eines HF-Generators kann durch Frequenzvervielfachung z.B. an einer Varactor-Diode auf das erforderliche Niveau angehoben werden. Man kann jedoch auch das Reaktionsgemisch innerhalb eines elektrischen Resonanzkörpers (z. B. Topfkreis oder Hohlleiter) anbringen, der durch den Generator zu stehenden Schwingungen angeregt wird. Weiterhin ist es möglich, ein langgestrecktes, dünnes Reaktionsgefäß (z. B. Kapillare) zu verwenden, das von dem Reaktionsgemisch mit langsamer Fließgeschwindigkeit durchströmt wird. Entlang der Achse dieses Reaktionsgefäßes wird eine stehende Welle erzeugt, die in regelmäßigen Abständen Knotenpunkte bildet, an denen die Intensität der elektrischen Wellen sich konzentriert, während dazu um 90° phasenverschoben Bereiche existieren, in denen sich die Wellen komplett auslöschen. Das Reaktionsgemisch wird während des Passierens dieser Stellen regelmäßig aufgeschmolzen. Durch geschickte Wahl der Parameter (lineare Strömungsgeschwindigkeit im Verhältnis zur Wellenlänge) kann dies mit den Zyklen der PCR synchronisiert werden. Es kommt somit zu einer kontinuierlichen Amplifikation entlang des Reaktionsgefäßes. Sowohl die Millimeterwellenkomponenten als auch die Kapillarstrukturen als ausgebildete Reaktionskammern können unschwer mikrosystemtechnisch hergestellt werden, was eine Integration der Bauteile auf einem Modul besonders begünstigt.

Die Frequenz der elektromagnetischen Wellen ergibt sich gemäß der vorstehenden Ausführungen aus den radialen Resonanzschwingungen zwischen den Nukleotidsträngen. Im Normalfall liegt die Frequenz zwischen 30 und ca. 60 GHz. Gegebenenfalls kann es sinnvoll sein, bei mehreren Frequenzen in diesem Bereich gleichzeitig einzustrahlen. Die (zusätzliche) Einstrahlung von völlig anderen Frequenzen kann unter Umständen nützlich sein, um Quervernetzungen bei komplizierten Tertiärstrukturen der DNA aufzulösen und dadurch die Amplifikation des DNA-Abschnittes zu erleichtern. Die Polymerase benötigt zum optimalen Arbeiten in der Regel eine Temperatur die über der Zimmertemperatur liegt (bei den heute gebräuchlichen Taq-Polymerasen ca. 72 °C). Durch dosiertes (zusätzliches) Einstrahlen bei einer Resonanzfrequenz des Lösungsmittels (in der Regel salzhaltiger wäßriger Puffer) kann die Temperatur des Reaktionsgemisches erhöht werden. Es entfällt somit auch eine zusätzliche Heizquelle.

Die Erfindung kann auch im Rahmen der in-situ PCR vorteilhaft verwendet werden. Hierbei wird beispielsweise ein Gewebeschnitt mit den PCR Reagentien versetzt, wobei zumindest ein Primer eine Markierung trägt. Mit Markierung sind jegliche Art von meß- oder färbetechnisch wirksame Markierungsatome oder Markierungsmoleküle gemeint. Dann wird der Gewebeschnitt dem o.g. Reaktionszyklus ausgesetzt mit dem Ergebnis, daß Nukleinsäuren mit sehr hoher Empfindlichkeit und lateraler Auflösung detektierbar sind. Mit der Erfindung wird erreicht, daß eine Austrocknung eines Gewebeschnitts und/oder eine Denaturierung von SToffen darin aufgrund der geringeren Aufheizung zuverlässig verhindert werden kann, und zwar unter Vermeidung der ansonsten hierfür erforderlichen Maßnahmen.

Folgend wird die Erfindung anhand von lediglich Ausführungsbeispielen darstellenden Figuren näher erläutert.

Es zeigen:
Fig. 1a: eine Darstellung eines apparativen Aufbaus zur Bestimmung von für die Trennung der Nukleinsäure Stränge einer doppelsträngigen Nukleinsäure Frequenzen,
Fig. 1b: eine Darstellung eines apparativen Aufbaus zur Verfolgung der Trennung der Nukleinsäure Stränge mittels UV-Transmission,
Fig. 1c: eine schematische Darstellung der geometrischen Zusammenhänge für eine erfindungsgemäße Vorrichtung,
Fig. 1d: einen apparativen Aufbau zur Durchführung des erfindungsgemäßen Verfahrens,
Fig. 1e: eine zum Gegenstand der Fig. 1d alternative Ausführungsform,
Fig. 1f: eine zu den Gegenständen der Figuren 1d und 1e weiterhin alternative Ausführungsform
Fig. 2: Dämpfungsmessungen von Puffer und NaCl-Lösung über Wasser,
Fig. 3: Dämpfungsmessungen verschiedener hochmolekularer DNA über Puffer,
Fig. 4: Dämpfungsmessungen verschiedener kurzer DNA über Puffer,
Fig. 5: Konzentrationsabhängigkeit der Dämpfung über Puffer für doppelsträngige DNA,
Fig. 6: Zeitabhängigkeit der UV-Absorption bei für doppelsträngiger DNA spezifischer UV-Frequenz bei Millimeterwelleneinstrahlung zur Beobachtung des hypochromatischen Effekts,
Fig. 7: eine Darstellung entsprechend Fig. 6 mit Rekombination nach Millimeterwellenabschaltung und
Fig. 8: eine Darstellung entsprechend Fig. 7, jedoch mit zusätzlicher Zugabe doppelsträngiger DNA während der Millimeterwelleneinstrahlung zum Zeitpunkt t = 300s.

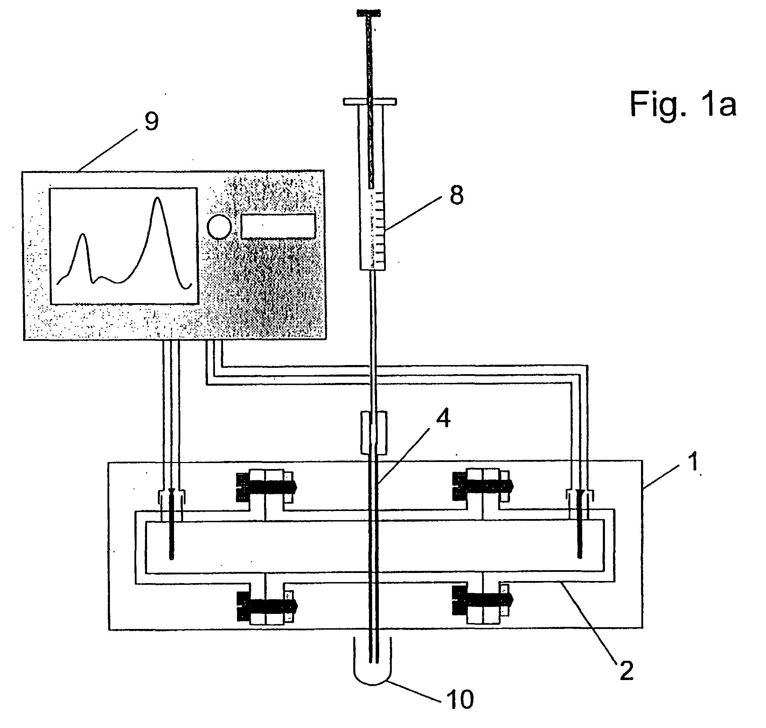

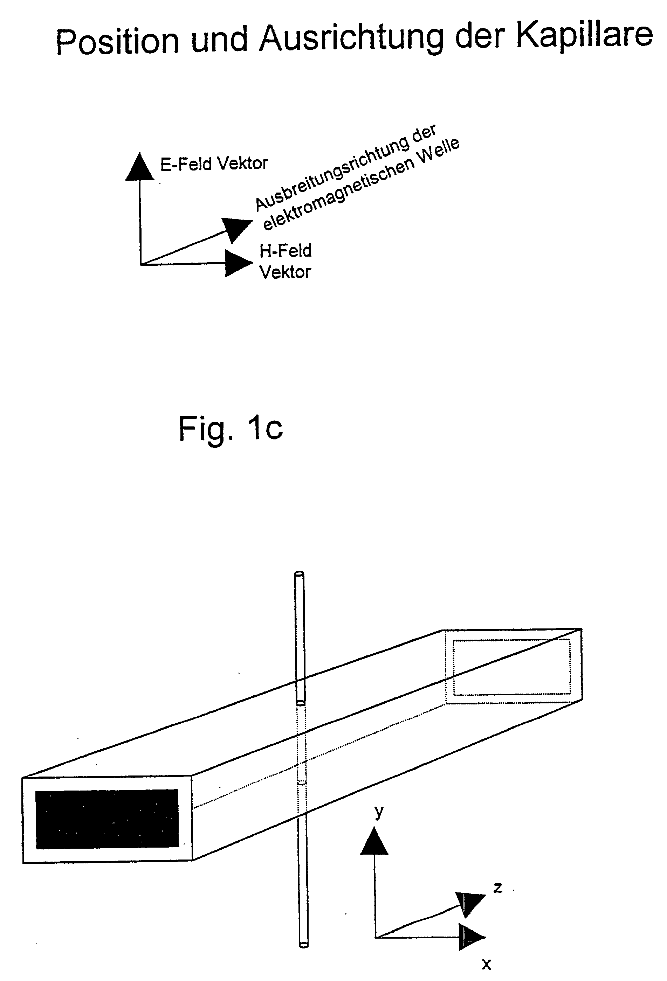

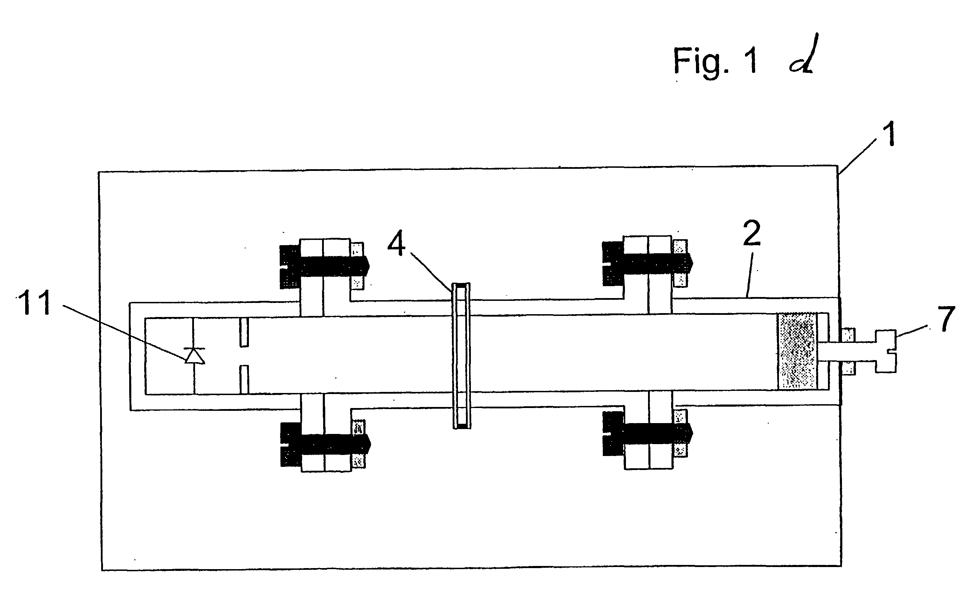

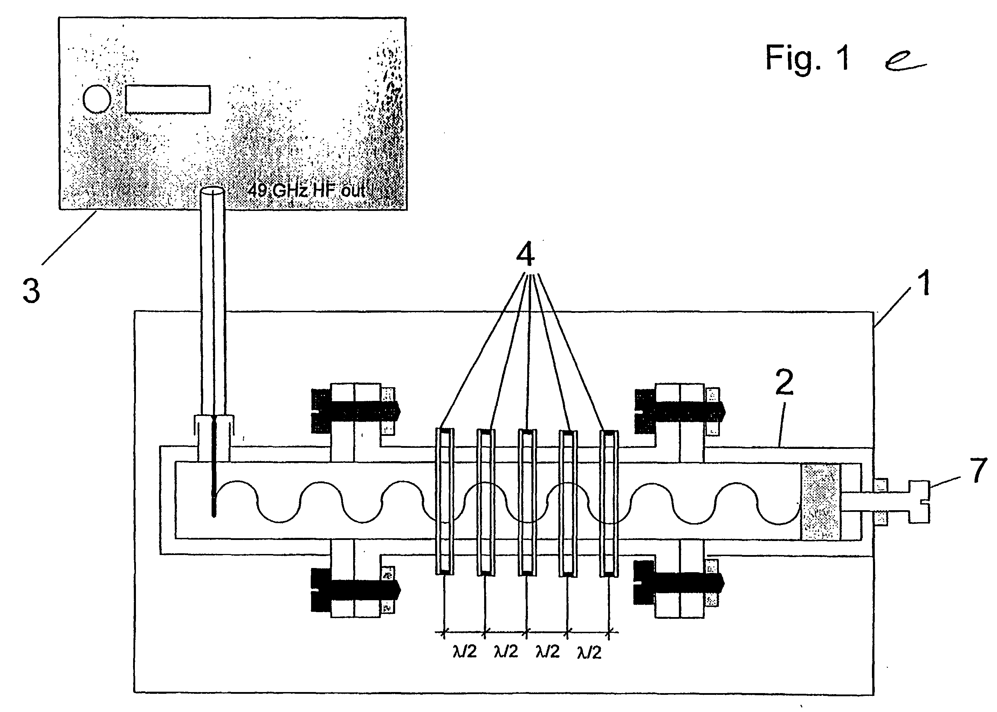

In der Fig. 1a erkennt man ein Gehäuse 1, in welchem ein Hohlleiter 2 (80mm langes Stück WR28 Hohlleiter mit Standardflanschen für Ka Band, WR19 Hohlleiter für U Band) eingerichtet ist. Der Hohlleiter 2 wird mittels eines HF-Generators auf einer einstellbaren Frequenz angeregt. Hierzu ist im Rahmen des Hohlleiters 2 eine Abstrahlantenne eingerichtet. Weiterhin ist eine Empfangsantenne vorgesehen, welche ebenso wie die Abstrahlantenne mit einem Spektrumanalyzer 9 verbunden ist. Durch das Innere des Hohlleiters 2 ist eine Kapillare 4 Glas, Länge 80mm, Innendurchmesser 0,7mm, Außendurchmesser 1,6mm) geführt, durch welche mittels einer Saugvorrichtung 8 Flüssigkeit aus einem Vorratsgefäß 10 angesaugt und beispielsweise auf einen Flussigkeitsspiegel von 10mm oberhalb der Oberkante des Hohlleiters 2 eingestellt werden kann (vertikale Anordnung der Kapillare). Mit diesem Aufbau wurden die folgend beschriebenen Dämpfungsmessungen durchgeführt, und zwar bei Frequenzen im Bereich von 40 bis 60 GHz.

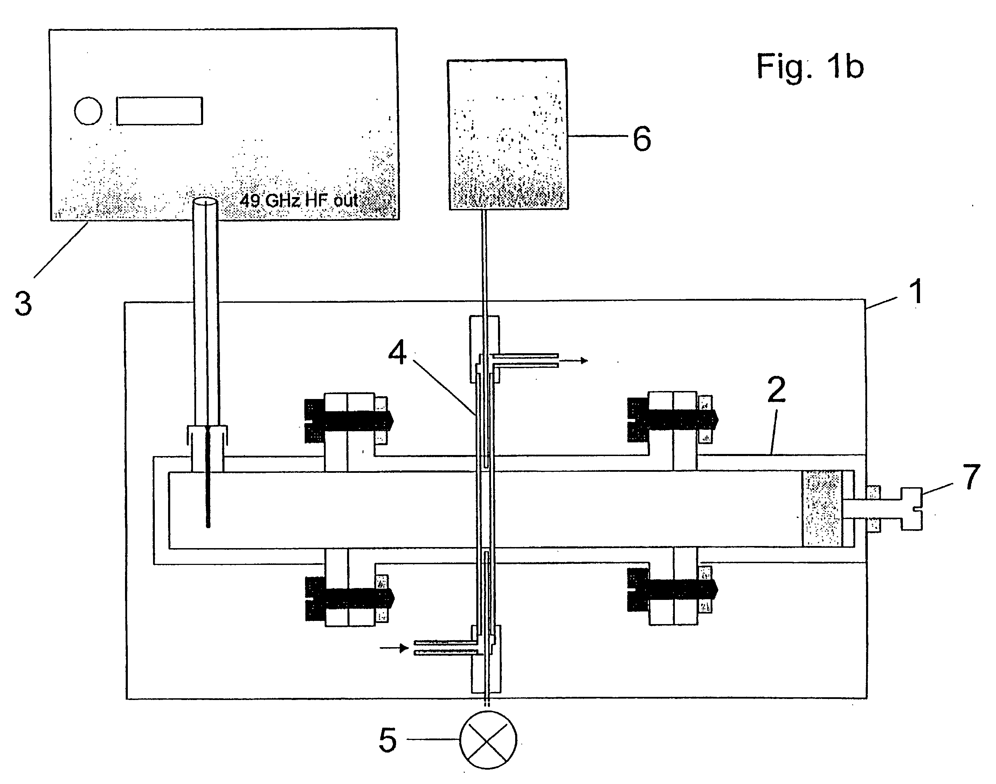

In der Figur 1b ist ein Aufbau zur Beobachtung des hypochromatischen Effekts dargestellt. Man erkennt als wesentliche Elemente einen Hohlleiter 2, einen auf eine geeignete Frequenz eingestellten HF-Generator 3 mit Abstrahlantenne und eine Kapillare 4 (mit zu untersuchender Flüssigkeit. Im Rahmen des Hohlleiters 2 ist ein Anpassungselement 7 zur Anpassung an die eingestrahlte Hochfrequenz vorgesehen. Weiterhin sind eine UV-Lichtquelle 5 ( 200 - 1000nm) sowie ein UV-Detektor 6 (spektrale Selektivität im Wellenlängenberich von 250 - 260 nm) eingerichtet, wobei die Anordnung so getroffen ist, daß die Kapillare in Ihrer Längsrichtung durchstrahlt wird. Die UV-Transmissionsmessungen erfolgten bei 260 nm und einer HF-Frequenz von 49 GHz. Mit einer Anordnung nach Fig. 1b lassen sich bei 20mW Sendeleistung 50% aller eingesetzten doppelsträngigen Nukleinsäuremoleküle innerhalb von 30s in einzelsträngige Nukleinsäuremoleküle aufspalten.

Die Fig. 1c zeigt eine zweckmäßige Ausrichtung von Kapillare 4 und Hohlleiter 2 bzw. elektrischem Feldvektor zueinander für einen geschlossenen Reaktor. Man erkennt, daß die Längserstreckung der Kapillaren parallel zum Vektor des elektrischen Feldes der (stehenden) elektromagnetischen Welle verlaufen sollte.

In der Figur 1d ist eine einfache Ausführungsform einer für PCR geeigneten Apparatur gezeigt. Man erkennt als wesentliche Elemente den Hohlleiter 2, das Anpassungselement 7 und die Kapillare 4. Die Kapillare 4 ist beidseitig verschlossen. Die Erzeugung der Hochfrequenz erfolgt hier durch eine Gunn-Diode 11.

In der Figur 1e ist ein Aufbau dargestellt, mit welchem Reaktionen in einer Mehrzahl von Kapillaren 4 durchgeführt werden können. Hierbei ist zu beachten, daß jede Kapillare in einem Maximum des elektrischen Feldvektors der stehenden Welle angeordnet ist, wie durch die schematisch angedeutete Welle verdeutlicht.

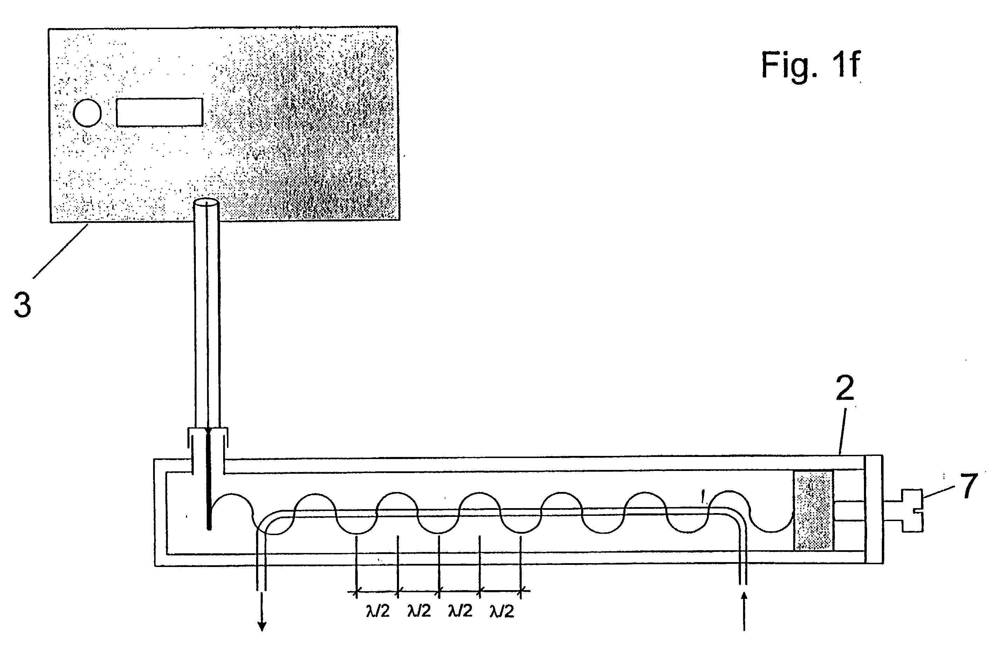

Die Figur 1f zeigt eine Ausführungsform von selbstständig erfinderischer Bedeutung. Hierbei ist ein Reaktionsabschnitt der Kapillare 4 in Längsrichtung des Hohlleiters 2 und orthogonal zum elektrischen Feldvektor und in Richtung der stehenden Welle verlaufend angeordnet. Das Reaktionsgemisch durchströmt dabei die Kapillare 4 mit einer definierten Strömungsgeschwindigkeit. Im Zuge der Durchströmung findet dann an Stellen hoher elektrischer Feldstärke die Trennung der doppelsträngigen Nukleinsäure in Einzelstränge statt, während in Bereichen kleiner elektrischer Feldstärke die Hybridisierung und Polymerasekatalysierte Anlagerung von Nukleotiden erfolgt. Im Zuge des Durchströmens wird also eine Mehrzahl von Amplifikationszyklen durchlaufen, wobei die Anzahl der Zyklen durch n = 2l / λ (n= Anzahl, l = Länge der Kapillare in Wellenrichtung, λ=Wellenlänge) gegeben ist. Die Strömungsgeschwindigkeit läßt sich dabei ohne weiteres in Verbindung mit der (konstant gehaltenen) Temperatur im Hinblick auf maximale Gesamtamplifikation optimieren.

Bei den mit der Apparatur nach Fig. 1a durchgeführten Dämpfungsmessungen wurde zunächst die Dämpfung, i.e. Absorption in Abhängigkeit von der Frequenz des Mediums gegenüber Wasser bestimmt. Diese Dämpfungskurven wurden dann als Bezugsgröße für die Bestimmung der Dämpfung bei Einbringungen einer DNA in das Medium genommen.'Auf diese Weise erhält man ein Millimeterwellen-Absortionsspektrum, welches von Mediumartefakten befreit ist.

Bei den UV-Absortionsmessungen ist die gewählte UV-Wellenlänge spezifisch für einzelsträngige DNA (hyperchromatischer Effekt). Eine hohe Transmission steht also für eine geringe Konzentration an einzelsträngiger DNA. Eine niedrige Transmission zeigt dagegen das Vorliegen einzelsträngiger DNA an.

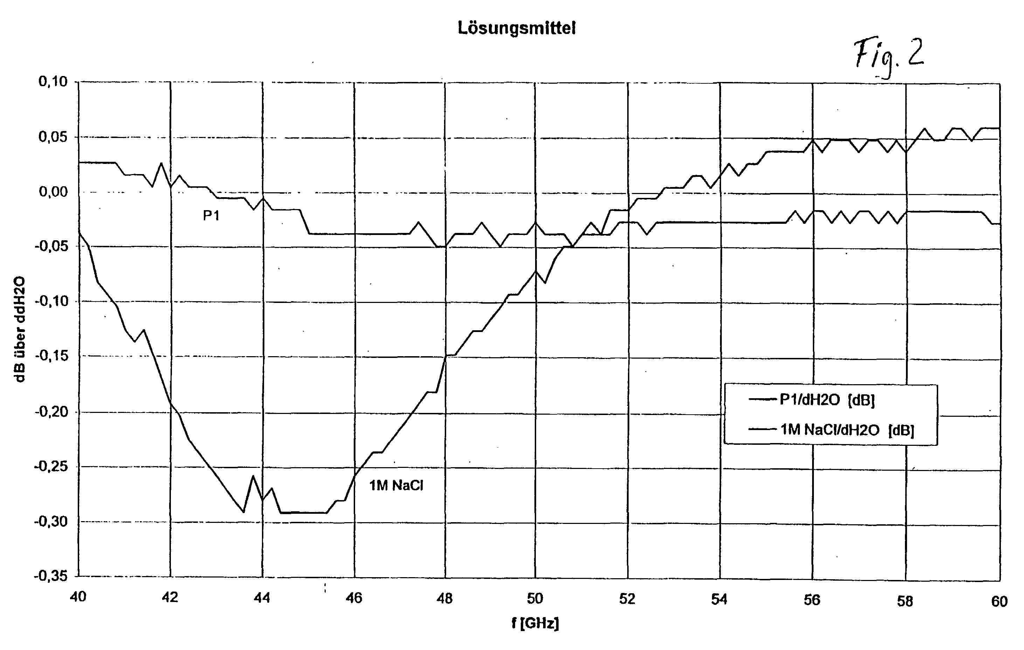

In der Figur 2 erkennt man den Dämpfungsverlauf eines Puffers der Zusammensetzung 9g/l NaCl, 50mM Tris/HCl, pH 8, in 1l ddH₂O sowie von 1M NaCl/dH₂O (d steht für destilliert). Während bei der NaCl-Lösung ein starkes Minimum bei ca. 45 GHz beobachtet wird, ist der spektrale Verlauf für den Puffer nahezu waagerecht. Der Puffer oder die NaCl wurde in allen folgenden Experimenten unverändert verwendet.

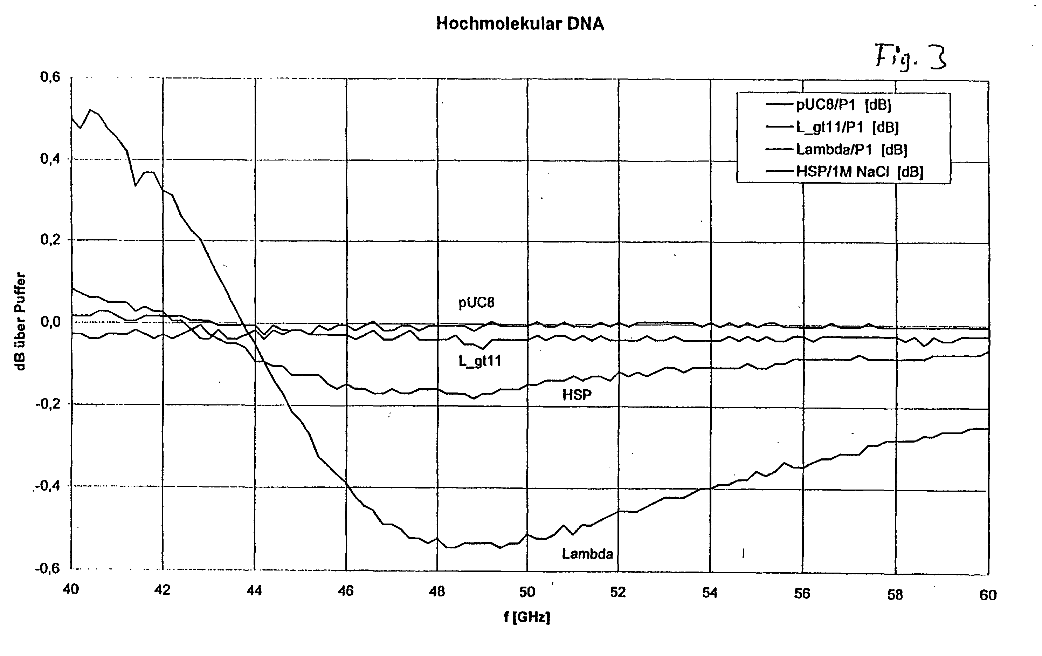

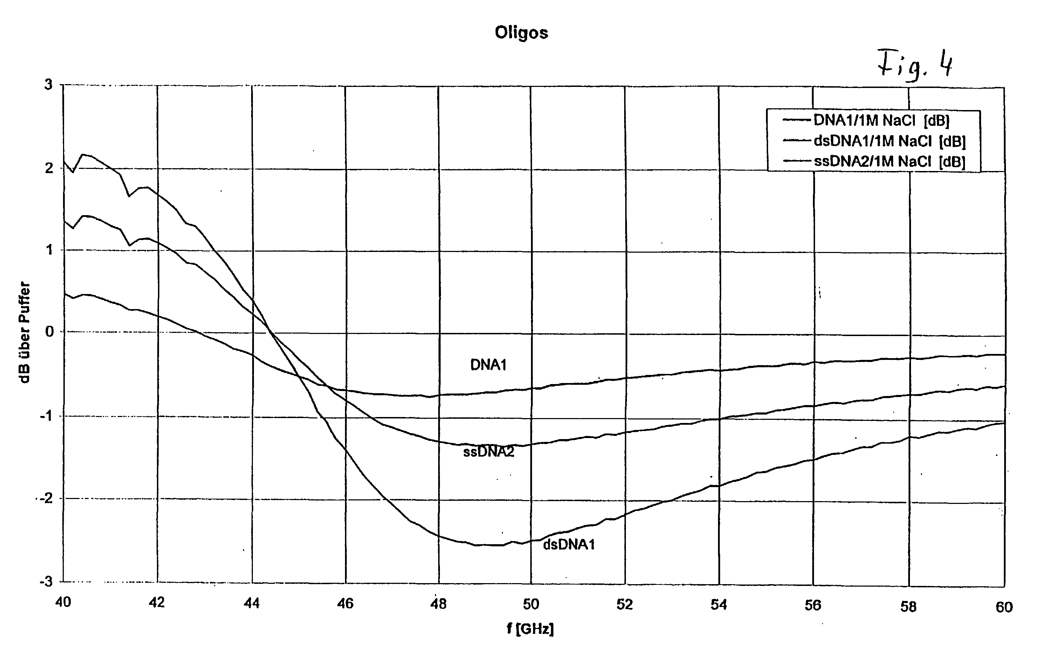

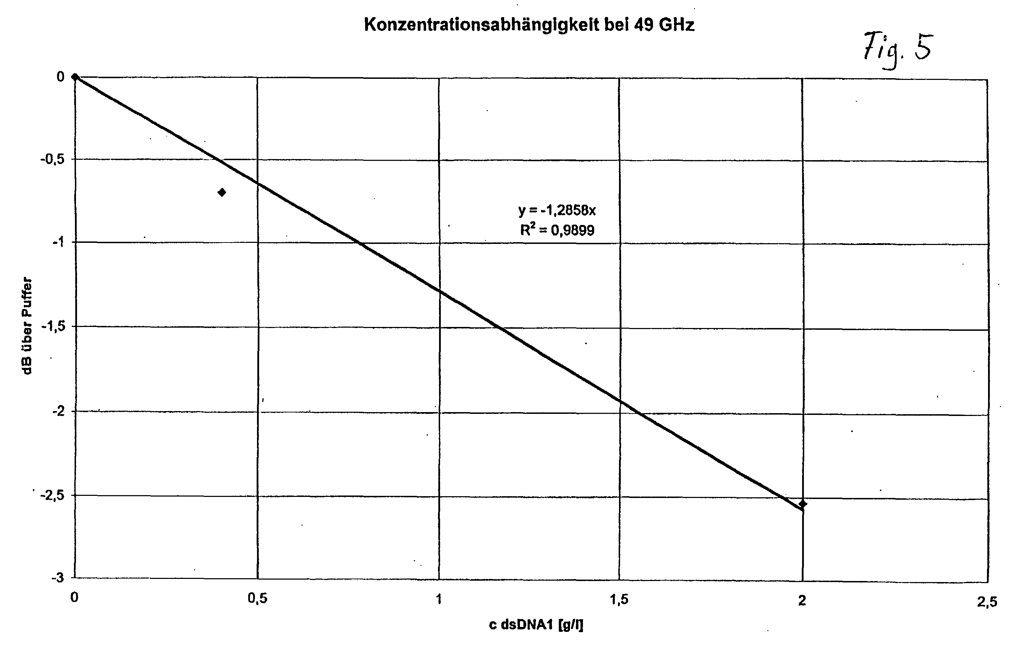

In der Figur 3 sind die Dämpfungen verschiedener hochmolekularer DNA dargestellt (für eine Aufstellung aller hier und folgend verwendeter DNA Proben siehe Tab.1). Man erkennt, daß jedenfalls bei vergleichsweise kurzer DNA ein charakterisstisches Minimum im Bereich von 47 bis 51 GHz auftritt; höchstwahrscheinlich eine Frequenz, bei welcher die DNA Moleküle jedenfalls teilweise unmittelbar angeregt werden. Die Figur 4 bestätigt dies insbesondere auch in der Gegenüberstellung der Spektren doppelsträngiger DNA mit der gleichen, jedoch einzelsträngigen DNA. Die Figur 5 belegt die Konzentrationsabhängigkeit für die doppelsträngige DNA 1.

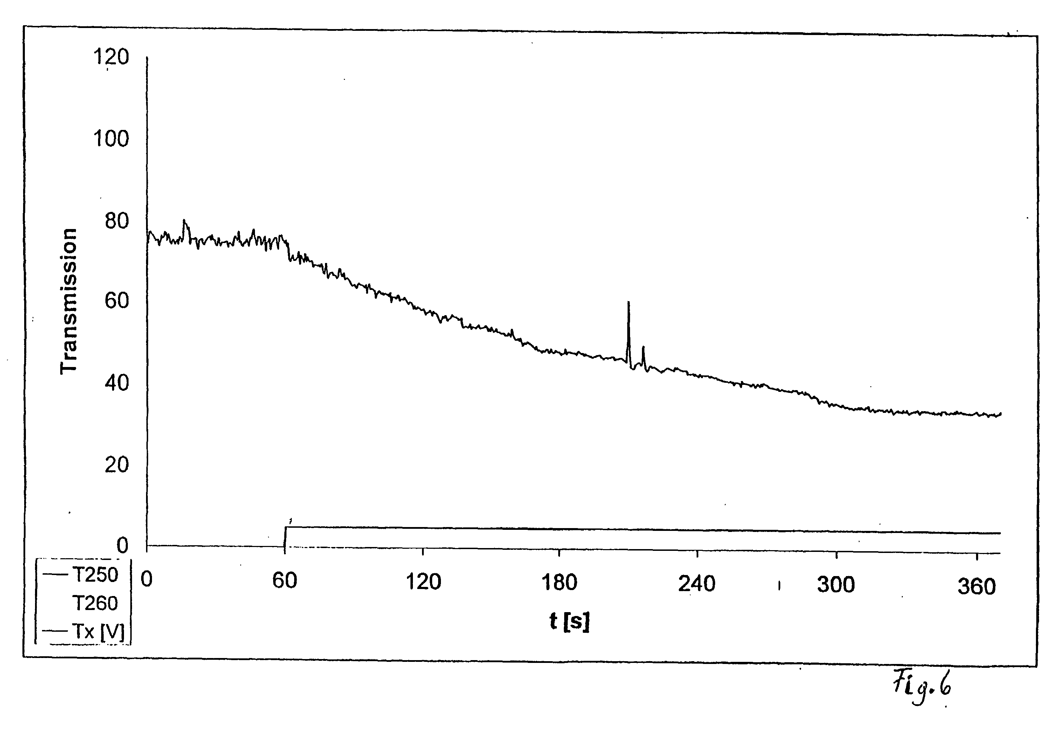

In den Versuchen der Figuren 6 bis 8 wurde bei t=0 eine Lösung der doppelsträngigen DNA 1 des Herstellers Sigma mit der Konzentration 10mM eingesetzt. In unteren Bereich der Diagramme ist der Verlauf der Betriebspannung des HF-Generators 3 dargestellt. Eine Betriebspannung von 0 steht für ausgeschalteten HF-Generator 3. Eine Betriebspannung von 5V steht für eingeschalteten HF-Generator 3.

In der Figur 6 erkennt bei kleinen Zeiten und ausgeschaltetem HF-Generator eine hohe und konstante Transmission aufgrund des Vorliegens doppelsträngige DNA. Zeitgleich mit der Anschaltung des HF-Generators 3 beginnt die Transmission jedoch abzunehmen, was auf der Abnahme der Konzentration doppelsträngiger DNA beruht. Die "Spitzen" beruhen auf Emissionsartefakten der UV-Quelle.

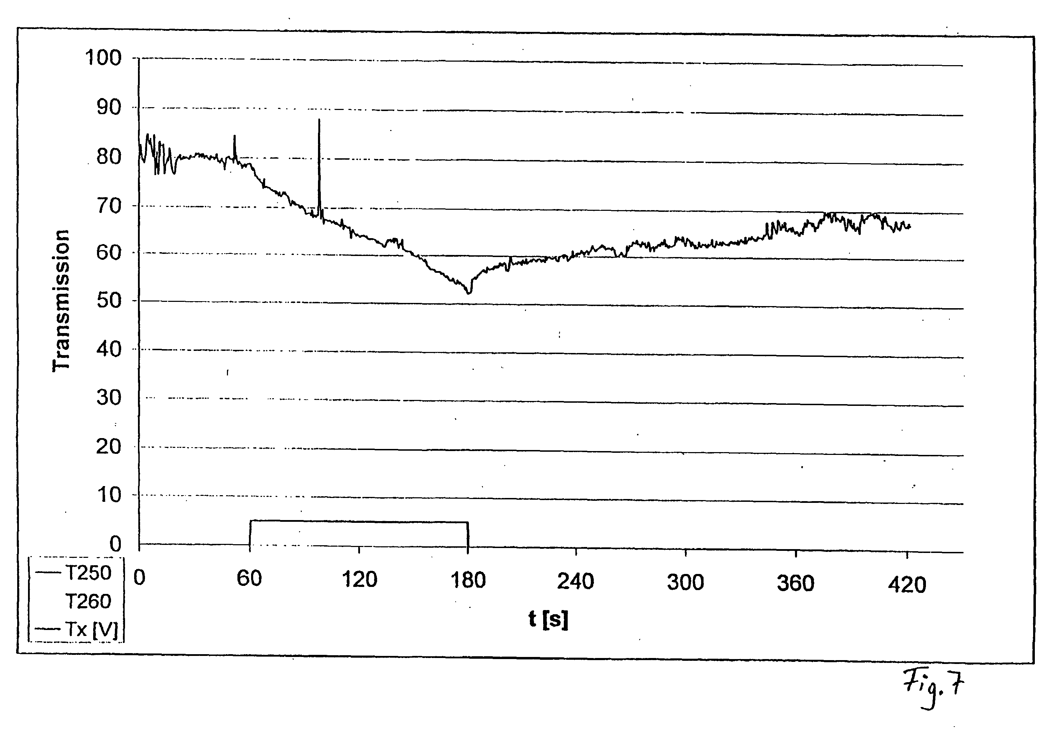

In dem Versuch der Figur 7 wurde verifiziert, daß die Abnahme der Konzentration doppelsträngiger DNA tatsächlich mit der Bildung von einzelsträngiger DNA korreliert ist. Denn nach dem Abschalten des HF-Generators erkennt man, daß die Transmission wieder ansteigt; das Ergebnis der Rekombination der einzelnen Stränge zur doppelsträngigen DNA.

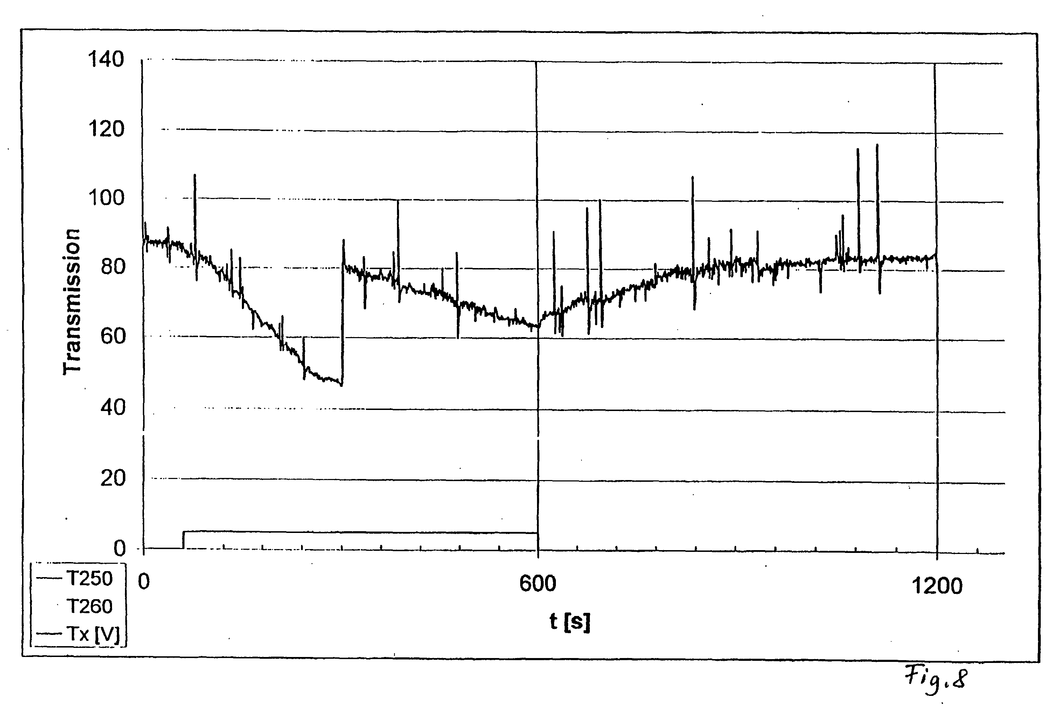

In dem Versuch der Figur 8 wurde während der Anschaltdauer des HF-Generators 3 doppelsträngige DNA nachgegeben mit dem Ergebnis, daß intermediär die Transmission wieder ansteigt.

Eine Vorrichtung durch Durchführung des erfindungsgemäßen in vitro PCR-Verfahrens ist im Prinzip wie im Stand der Technik aufgebaut. Der wesentliche Unterschied besteht darin, daß zusätzlich oder anstelle der konventionellen Einrichtungen zur Erwärmung auf 95 °C eine Einrichtung zur Einstrahlung elektromagnetischer Wellen in die Reaktionskammer eingerichtet ist. Bezüglich Details hierzu darf auf die Figuren 1a bis 1f verwiesen werden, die weiteren Komponenten entsprechen dem üblichen Aufbau und brauchen daher nicht näher erläutert werden. Es versteht sich, daß die UV-Quelle und der UV-Detektor für die Durchführung der PCR entbehrlich sind.

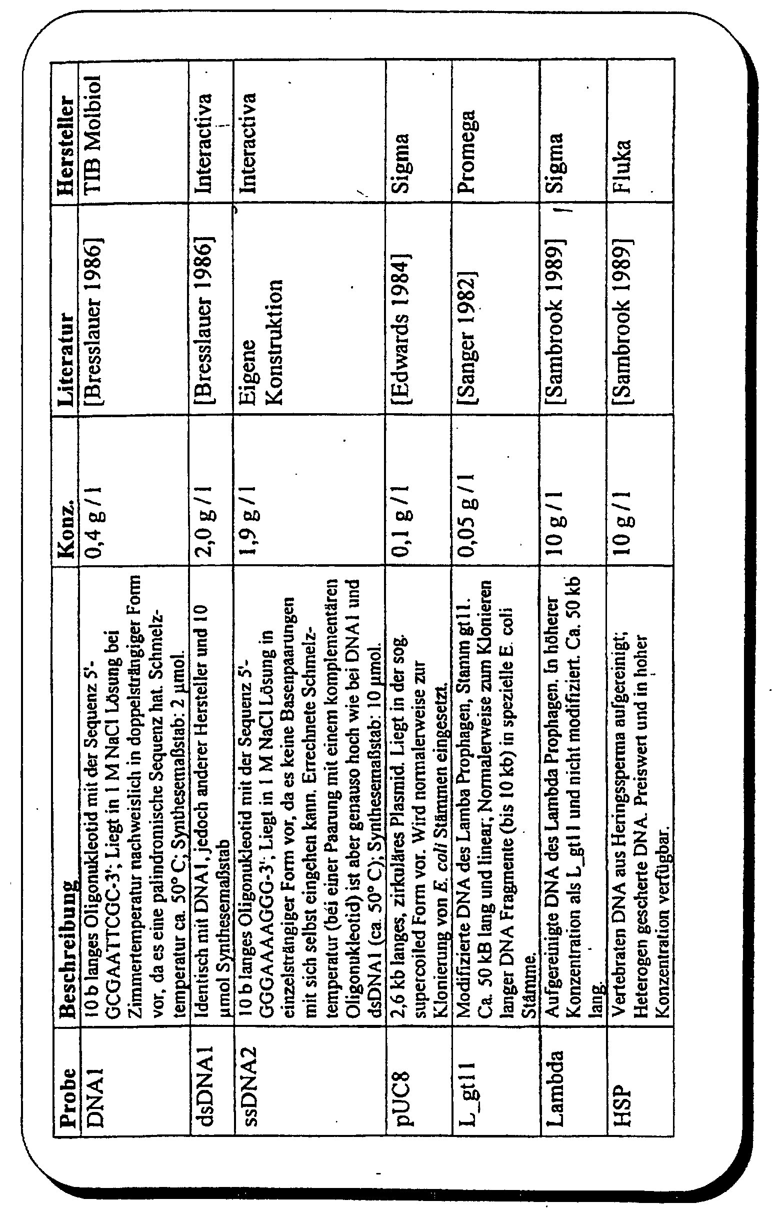

## Patentansprüche

1. Verfahren zur Auftrennung von in Lösung befindlichen doppelsträngigen Nukleinsäuren in einzelsträngige Nukleinsäuren,
**dadurch gekennzeichnet,**
**daß** die Zerlegung der Nukleinsäuren in Einzelstränge durch Einstrahlung von elektromagnetischen Wellen durchgeführt wird,
wobei die Frequenz und die Intensität der elektromagnetischen Wellen mit der Maßgabe ausgewählt sind, daß die Wasserstoffbrückenbindungen zwischen Nukleotiden einer doppelsträngigen Nukleinsäure durch unmittelbare Wechselwirkung der elektromagnetischen Strahlung mit der doppelsträngigen Nukleinsäure gelöst werden.

2. Verfahren nach Anspruch 1, **dadurch gekennzeichnet, daß** die Freqenz der elektromagnetischen Strahlung im Bereich von 10GHz bis 2THz, vorzugsweise 30 bis 79 GHz, eingestellt ist mit der Maßgabe, daß die eingestellte Frequenz zumindest teilweise solche Schwingungszustände der Nukleinsäure anregt, welche in einer Trennung ausschließ- lich der Wasserstoffbrückenbindungen resultieren, nicht jedoch Bindungen einer Polymerase spaltet oder zu einer Temperaturerhöhung eines Mediums führt.

3. Verwendung eines Verfahrens nach Anspruch 1 oder 2 in einem Verfahren zur in vitro Amplifikation von Nukleinsäuren mit den folgenden Verfahrenstufen: a) Zerlegung der Nukleinsäuren in Einzelstränge, b) Anhybridisierung von Primern an die Einzelstränge aus Stufe a), c) Verlängerung der in Stufe b) anhybridisierten Primer durch (Desoxy-)Ribonukleosidtriphosphate mittels Einsatz einer Polymerase d) Rückführung der in Stufe c) erhaltenen Nukleinsäuren in die Stufe a), wobei die Stufen a) - d) so oft wiederholt werden, bis ein vorgegebener Amplifikationsfaktor erreicht ist und wobei die elektromagnetische Strahlung in Stufe a) eingestrahlt wird.

4. Verwendung nach Anspruch 3, **dadurch gekennzeichnet, daß** auf Nukleinsäure-Synthese-Geschwindigkeit optimierte Polymerasen in Stufe b) eingesetzt werden.

5. Vorrichtung zur Durchführung eines Verfahrens nach einem der Ansprüche 1 bis 4 mit einer Reaktionskammer zur Aufnahme einer Lösung mit Nukleinsäuren, mit einer Vorrichtung zur Erzeugung elektromagnetischer Wellen sowie mit einem Antennenelement zur Abstrahlung der elektromagnetischen Strahlung, wobei das Antennenelement unmittelbar bei der Reaktionskammer angeordnet ist und wobei zumindest eine Betriebsfrequenz der Vorrichtung zur Erzeugung elektromagnetischer Strahlung im Bereich von 10 bis 250 GHz und/oder von 0,5 bis 2,0 THz liegt.

6. Vorrichtung nach Anspruch 5, wobei die Reaktionskammer innerhalb eines als Antennenelement funktionierenden elektrischen Resonanzkörpers, insbesondere eines Topfkreises oder Hohlleiters, angeordnet ist.

7. Vorrichtung nach Anspruch 5 oder 6, wobei die Reaktionskammer als Rührkessel-Reaktor ausgebildet ist.

8. Vorrichtung nach Anspruch 5 oder 6, wobei die Reaktionskammer als Rohrreaktor ausgebildet ist und wobei im Inneren des Rohrreaktors und in Richtung der Längserstreckung des Rohrreaktors stehende elektromagnetische Wellen erzeugbar sind.

## Claims

1. A method for fractionating double-stranded nucleic acids in solutions in order to obtain single-stranded nucleic acids, **characterized in that** the fractionation of the nucleic acids into single strands is achieved by electro-magnetic radiation, wherein the frequency and intensity of the electro-magnetic waves is selected such that the hydrogen bonds between nucleotides of a double-stranded nucleic acid are broken by direct interaction of the electro-magnetic radiation with the double-stranded nucleic acid.

2. A method according to claim 1, **characterized in that** the frequency of the electro-magnetic radiation is adjusted within the range of 10 GHz to 2 THz, preferably 30 to 79 GHz, such that the adjusted frequency excites at least partially such oscillation states of the nucleic acid resulting in a breakage exclusively of the hydrogen bonds, but not fractionate bonds of a polymerase or lead to a temperature increase of a medium.

3. An application of the method according to claim 1 or 2 in a method for the in-vitro amplification of nucleic acids, comprising the following steps: a) fractionation of the nucleic acid into single strands, b) hybridization of primers to the single strands of step a), c) extension of the primers hybridized in step b) by (desoxy-) ribonucleoside triphosphates by means of a polymerase, d) return of the nucleic acids obtained in step c) to step a), with steps a) to d) being repeated so many times, until a given amplification factor is achieved, and the electro-magnetic radiation being irradiated in step a).

4. An application according to claim 3, **characterized in that** polymerases optimized for the nucleic acid synthesis speed are used in step b).

5. A device for carrying-out a method according to one of claims 1 to 4, comprising a reaction chamber for receiving a solution with nucleic acids, a device for generating electro-magnetic waves and an antenna element for irradiating the electro-magnetic radiation, said antenna element being located immediately at the reaction chamber, and at least one operating frequency of the device for generating electro-magnetic radiation is in the range of 10 to 250 GHz and/or 0.5 to 2.0 THz.

6. A device according to claim 5, wherein said reaction chamber is disposed within a electrical resonance body, such as a cavity resonator or a hollow conductor and operating as an antenna element.

7. A device according to claim 5 or 6, wherein said reaction chamber is adapted as a stirrer vessel reactor.

8. A device according to claim 5 or 6, wherein said reaction chamber is adapted as a tube reactor, and wherein inside the tube reactor and in a direction of the longitudinal extension of the tube reactor standing electro-magnetic waves can be generated.

## Revendications

1. Procédé de séparation d'acides nucléiques à double brin en solution permettant d'obtenir des acides nucléiques à un seul brin, **caractérisé en ce que** la séparation des acides nucléiques à des seuls brins est exécutée par l'irradiation d'ondes électromagnétiques, la fréquence et l'intensité des ondes électromagnétiques étant choisies de telle manière, que les liaisons hydrogène entre les nucléotides d'une acide nucléique à double brin soient séparées par l'interaction immédiate de la radiation électromagnétique avec l'acide nucléique à double brin.

2. Procédé selon la revendication 1, **caractérisé en ce que** la fréquence de la radiation électromagnétique est réglée à une gamme de 10 GHz à 2 THz, préférentiellement 30 à 79 GHz, de sorte que la fréquence réglée excite au moins partiellement de tels états vibratoires résultant en une séparation uniquement des liaisons hydrogène, mais ne séparant pas les liaisons d'une polymérase ou ne menant pas à une augmentation de la température d'un milieu.

3. Utilisation d'un procédé selon la revendication 1 ou 2 dans un procédé pour l'amplification in vitro d'acides nucléiques, comprenant les étapes suivantes: a) décomposition des acides nucléiques en seuls brins, b) hybridation de primers aux seuls brins de l'étape a), c) prolongation des primers hybridés en étape b) par des (desoxy-)ribo-nucleoside-phosphates par l'utilisation d'une polymérase, d) retour des acides nucléiques obtenus en étape c) dans l'étape a), les étapes a) à d) étant répétées jusqu'à ce qu'une amplification prédéfinie soit atteinte, et la radiation électromagnétique étant irradiée en étape a).

4. Utilisation selon la revendication 3, **caractérisée en ce que** des polymérases optimalisées en ce qui concerne la vitesse de la synthèse de l'acide nucléique sont utilisées.

5. Dispositif pour l'exécution d'un procédé selon une des revendications 1 à 4, comprenant une chambre de réaction pour recevoir une solution avec des acides nucléiques, un dispositif pour générer des ondes électromagnétiques et un élément d'antenne pour l'irradiation de la radiation électromagnétique, cet élément d'antenne étant positionné immédiatement à la chambre de réaction et au moins une fréquence d'opération du dispositif pour générer la radiation électromagnétique étant dans la gamme de 10 à 250 GHz et/ou de 0,5 à 2,0 THz.

6. Dispositif selon la revendication 5, dans lequel cette chambre de réaction est positionnée au-dedans d'un élément de résonance, en particulier d'une cavité résonante ou d'une âme creuse fonctionnant comme élément d'antenne.

7. Dispositif selon la revendication 5 ou 6, dans lequel cette chambre de réaction est configurée comme un réacteur agitateur.

8. Dispositif selon la revendication 5 ou 6, dans lequel cette chambre de réaction est configurée comme un réacteur tubulaire et dans lequel des ondes électromagnétiques stationnaires peuvent être générées en direction de l'étendue longitudinale du réacteur tubulaire.
